# EUROPEAN PATENT SPECIFICATION

(11) **EP 2 072 053 B1**
(45) Date of publication and mention of the grant of the patent: **07.11.2012**
(21) Application number: 07301736.0
(22) Date of filing: 21.12.2007
(51) Int. Cl.: A61K 35/74, A23L 1/30, A61P 1/00

(54) **Method for decreasing abdominal girth by administering a bifidobacterium bacteria**
Verfahren zur Verringerung des Bauchumfangs durch Verabreichung einer Bakterie der Gattung Bifidobacterium
Procédé pour diminuer la sangle abdominale en administrant des bactéries Bifidobacterium

(43) Date of publication of application: 24.06.2009
(73) Proprietor: Compagnie Gervais Danone, 75009 Paris (FR)
(72) Inventor: Goupil-Feuillerat, Nathalie, 91120, Palaiseau (FR); Guyonnet, Denis, 92300, LEVALLOIS PERRET (FR); Jakob, Stefan, 78730, SAINT ARNOULT-EN-YVELINES (FR)
(74) Representative: Cabinet Plasseraud

(56) References cited:
- GUYONNET D ET AL: "Effect of a fermented milk containing Bifidobacterium animalis DN-173 010 on the health-related quality of life and symptoms in irritable bowel syndrome in adults in primary care: a multicentre, randomized, double-blind, controlled trial" ALIMENTARY PHARMACOLOGY & THERAPEUTICS, vol. 26, no. 3, August 2007 (2007-08), pages 475-486, XP002479503 ISSN: 0269-2813
- O'MAHONY LIAM ET AL: "Lactobacillus and bifidobacterium in irritable bowel syndrome: symptom responses and relationship to cytokine profiles." GASTROENTEROLOGY MAR 2005, vol. 128, no. 3, March 2005 (2005-03), pages 541-551, XP002479504 ISSN: 0016-5085
- HOUGHTON LESLEY A ET AL: "Relationship of abdominal bloating to distention in irritable bowel syndrome and effect of bowel habit" GASTROENTEROLOGY, vol. 131, no. 4, October 2006 (2006-10), pages 1003-1010, XP005686001 ISSN: 0016-5085
- RENEE TANISHA: "Dannon's Activia Yogurt: Lose Weight and Feel Great", Yahoo! Voices , 17 April 2007 (2007-04-17), Retrieved from the Internet: URL:http://voices.yahoo.com/dannons-activi a-yogurt-lose-weight-feel-great-287207.htm l?cat=5 [retrieved on 2012-01-04]
- MARTEAU P ET AL: "Bifidobacterium animalis strain DN-173 010 shortens the colonic transit time in healthy women: A double-blind, randomized, controlled study", ALIMENTARY PHARMACOLOGY & THERAPEUTICS, BLACKWELL SCIENTIFIC PUBLICATIONS LTD., CAMBRIDGE, GB, vol. 16, no. 3, 1 March 2002 (2002-03-01), pages 587-593, XP002399114, ISSN: 0269-2813, DOI: 10.1046/J.1365-2036.2002.01188.X
- TAMURA N ET AL: "Alteration of fecal bacterial flora following oral administration of bifidobacterial preparation.", GASTROENTEROLOGIA JAPONICA FEB 1983 LNKD- PUBMED:6832549, vol. 18, no. 1, February 1983 (1983-02), pages 47-55, ISSN: 0435-1339
- "Activia", Wikipedia , 3 January 2012 (2012-01-03), Retrieved from the Internet: URL:http://en.wikipedia.org/w/index.php?ti tle=Activia&oldid=469403809 [retrieved on 2012-01-04]

## Description

The present invention relates to Bifidobacterium animalis for use in decreasing abdominal girth in an IBS subject having or subjected to abdominal girth increase, wherein at least 1 x 10⁹ cfu of said *Bifidobacterium animalis* is administered per day to said subject during at least 15 days. The present invention also relates to a non therapeutic method for decreasing abdominal girth in a healthy subject, comprising the step of administering to said subject at least about 1 x10⁹ cfu of a bacteria chosen in the group of bacteria of the *Bifidobacterium animalis* per day.

Abdominal girth changes during the day in normal healthy individuals and seems to follow a particular pattern-namely that it increases as the day progresses, is more pronounced after meals and subsides at night.
Increase of abdominal girth, especially after meals, is, for a lot of people, an everyday or punctual discomfort that they would like to make disappear or at least to reduce.

Important increase of abdominal girth may also indicate functional bowel disorder like irritable bowel syndrome (IBS).

Irritable bowel syndrome (IBS) is a functional bowel disorder in which abdominal pain or discomfort is associated with defecation or change in bowel habit and with features or disordered defecation.

Patients can present with either constipation (IBS-C) or diarrhoea (IBS-D), or a mixture of both (IBS-M) or none of these three characteristics (IBS-U ; U means "Unsubtype") (1). IBS affects between 10% and 20% of the population (1-4), and results in significant work absenteeism and reduced quality of life (5-6). Although it accounts for nearly half of gastroenterology clinic referrals (7), the treatment available is far from adequate (1, 7).

Thus there is need for means to reduce partially or totally small increases of abdominal girth for healthy subject but also to reduce partially or totally more important increases of abdominal girth for IBS patients.

It has been shown that the use of certain probiotic preparations, containing bacteria such as propionic bacteria, lactobacilli and/or bifidobacteria, makes it possible to modify the flora in the colon of certain patients (8, 9, 9bis). Moreover, in both man and animal models, a number of probiotics have been shown to modify gastrointestinal contractility or excessive flatulence, meteorism, or abdominal pain (10).

Guyonnet et al., Alimentary Pharmacology & Therapeutics, 2007, discloses the effects of Bifidabacterium animalis DN-173 010 on the IBS. Marteau et al., Aliment Pharmacol Ther., 2002, discloses the use of *Bifidobacterium anima*/*is* DN-173 0 10 for shortening the colonic transit time in healthy women.

Surprisingly, it has been found that administration of at least about 1x 10⁹ cfu of Bifidobacterium animalis per day during at least 15 days can reduce significantly abdominal girth in a subject.

An object of the present invention is therefore *Bifidobacterium animalis* for use according to claim 1, and the non therapeutic method according to claim 14.

In a preferred embodiment of the invention said bacteria is administered in the non therapeutic method during at least 15 days.

The term "abdominal girth" is intended to mean the measurement of the distance around the abdomen at a specific point, usually at the level of the belly button (navel). Abdominal inductance plethysmography can be used to measure abdominal girth.
The measurement of the abdominal girth may also be helpful in determining objective abdominal distention.

For the comprehension of the invention, it has to be understood that increase of abdominal girth and bloating are not synonymous for the man state in the art, specialist in gastroenterology.
Until recently research into bloating and distention has been sparse and largely empirical as well as being based on the assumption that the two descriptors were describing the same phenomenon. Thus interpreting the data from older studies is difficult and even today patients and their physicians often use the terms synonymously. However, with the development of more objective ways of assessing it such as the gas challenge technique or abdominal inductance plethysmography, there is increasing evidence that bloating and distention may have different pathophysiological mechanisms.

While bloating is a subjective sensation measured by a questionnaire submitted to the patient, increase of abdominal girth can be measured by objective ways, such as abdominal inductance plethysmography. Numerous studies have shown that only approximately half of patients reporting the sensation of bloating exhibit objective abdominal distension beyond a 90% control range, as measured by the recently validated technique of Abdominal Inductance Plethysmography.

Sensory mechanisms appear to be more important in bloating and thus this symptom may be eased by drugs affecting this modality (for example, tricyclic antidepressants have been suggested to be tested (11). In contrast, abdominal distension have more mechanical basis and it has been suggested to test laxatives and prokitenics, which are a class of drugs used on the digestive system (e.g. Alosetron or Tegaserod) (11).
Thus it has become apparent that not all individuals who feel bloated necessarily exhibit an increase in abdominal girth. This has led that the term bloating should be used to describe the sensation of increased abdominal pressure and the descriptor distension and abdominal girth should only be used when there is an actual change in abdominal circumference (11).
Complex mechanisms which sometimes overlap seem to be involved in abdominal distension (11). Increase of abdominal girth is observed in both IBS-C and IBS-D, which are associated with opposite transit conditions, i.e. slow and rapid respectively (13). This is also supported by the fact that other mechanisms than transit are involved in the abdominal girth increase such as visceral sensation or gas handling as well as sex-related mechanisms (11).

The term "administering" is intended to mean "administering orally" i.e. that the subject will orally ingesting a bacteria according to the present invention or a composition comprising the bacteria according to the present invention, or "administering directly" i.e. that a bacteria according to the present invention or a composition comprising the bacteria according to the present invention will be directly administered in situ, in particular by coloscopy, or rectally via suppositories.

Oral administration of composition comprising the bacteria for use according to the present invention may be in the form of gelatin capsules, of capsules, of tablets, of powders, of granules or of oral solutions or suspensions.

In a preferred embodiment of the invention, said composition for use according to the invention is a food composition which can be used in the production of new foods or food ingredients as defined in EC Regulation No. 258/97, and in particular in the manufacture of functional foods. A food may be considered to be functional if it is demonstrated satisfactorily that it exerts a beneficial effect on one or more target functions in the organism, beyond the usual nutritional effects, improving the state of health and of well-being and/or reducing the risk of a disease (12).

In a preferred embodiment of the invention, said bacteria is administered in the form of a dairy product. In particular, the dairy product is a fermented dairy product and more particularly the fermented dairy product is a yoghourt.

Said composition may in particular constitute a probiotic packaged, for example, in the form of a capsule or a gelatin capsule.

In another preferred embodiment of the invention, said composition for use according to the invention is a pharmaceutical composition, also combined with a pharmaceutically acceptable carrier, which may comprise excipients.

Preferably, the pharmaceutical composition also comprises at least one other agent active against IBS.

The term "pharmaceutical composition" is intended to mean "drug" or "OTC (Over The Counter)".

According to the invention, the decrease of abdominal girth of the subject is at least about 1 cm.
Preferably, the decrease of abdominal girth of the subject is at least about 1,5 cm.

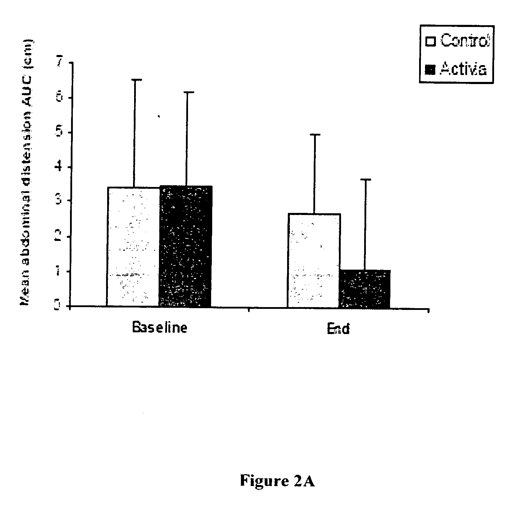

It is also possible to quantify the decrease of abdominal girth of the subject in percentage of decrease of the abdominal girth of the subject.
According to experiments done by the inventors (see "Examples" part), the mean values of abdominal girth in the subjects observed are about 81.5 cm in baseline conditions at the beginning of the day (i.e. morning) and the mean increase of abdominal girth during the day is 3,5 cm (figure 2A).

In an embodiment of the invention, the decrease of abdominal girth is a decrease in the relative maximum abdominal distension.

The term "relative decrease in maximum abdominal distension" is intended to mean "percentage of maximum abdominal distension reduction".
The "Maximum distension" or "maximum abdominal distension" or "maximum abdominal girth increase" is defined as the mean abdominal girth over one hour of recording at which girth was at its greatest.

For example, a patient having an abdominal girth of 81,5 cm in baseline conditions, and having a maximum abdominal girth increase of 6 cm, which would have a decrease of abdominal girth of 3 cm (i.e 3,7% of the abdominal girth) will show a decrease of the relative maximum abdominal distension of 50% (3cm/6cm).

Preferably, the decrease in the relative maximum abdominal distension is at least about 50%.

Moreover, it has to be noted that increases of this abdominal girth have been observed until 12 cm in some patients with IBS (13).

The bacteria is chosen in the group of bacteria of the *Bifidobacterium* animalis and is considered as a probiotic.

The term "probiotics" is intended to mean dietary supplements containing potentially beneficial bacteria or yeasts. According to the currently adopted definition by FAO/WHO, probiotics are: 'Live microorganisms which when administered in adequate amounts confer a health benefit on the host'. Lactic acid bacteria are the most common type of microbes used. Lactic acid bacteria have been used in the food industry for many years, because they are able to convert sugars (including lactose) and other carbohydrates into lactic acid. This not only provides the characteristic sour taste of fermented dairy foods such as yogurt, but also by lowering the pH may create fewer opportunities for spoilage organisms to grow, hence creating huge health benefits on preventing gastrointestinal infections. Strains of the genera *Lactobacillus* and *Bifidobacterium*, are the most widely used probiotic bacteria.
Probiotic bacterial cultures are intended to assist the body's naturally occurring gut flora to reestablish themselves. They are sometimes recommended by doctors, and, more frequently, by nutritionists, after a course of antibiotics, or as part of the treatment for gut related candidiasis. Claims are made that probiotics strengthen the immune system to combat allergies and other immunal diseases.

The bacteria is chosen in the group of *Bifidobacterium animalis.*

More preferably the bacteria is *B. animalis* CNCM I-2494.

The bacteria according to the present invention is a *Bifidobacterium animalis*.
More preferably the bacteria according to the present invention is a *Bifidobacterium animalis* deposited under the number I-2494 at CNCM on June 20, 2000. This strain is known under the code DN-173 010 and is protected, with its use as glycosylation modulator of intestinal cell surface, by European Patent EP 1 297 176.

In a preferred embodiment of the invention, about 1x 10¹⁰ cfu of bacteria per day is administered to the subject during at least 15 days, preferably at least 28 days.
More preferably, 1,25x 10¹⁰ cfu of bacteria is administered to the subject two times per day during at least 15 days, preferably at least 28 days.
According to the invention, the subject can be a child, an adult or an elderly, preferably an adult.
In a preferred embodiment of the invention the subject is a female.
Indeed it has been shown that female subjects has predisposition for increase of abdominal girth, abdominal distension and irritable bowel syndrome (1, 13).
In a preferred embodiment of the non therapeutic method, the subject has or is subjected to abdominal girth increase.

The purpose of said non therapeutic method is to decrease the abdominal girth of healthy subjects of the population, in particular after meals. Preferably, said non therapeutic method is to decrease the relative maximum distension of abdominal girth of said subjects.

According to a preferred embodiment of the invention, the composition for use according to the invention is for treating irritable bowel syndrome (IBS).

In gastroenterology, irritable bowel syndrome (IBS) or spastic colon is a functional bowel disorder characterized by abdominal pain and changes in bowel habits which are not associated with any abnormalities seen on routine clinical testing. It is fairly common and makes up 20-50% of visits to gastroenterologists. Lower abdominal pain, and bloating associated with alteration of bowel habits and abdominal discomfort relieved with defecation are the most frequent symptoms. The abdominal pain type is usually described in a patient as either constipation-predominant (IBS-C) or diarrhoea-predominant (IBS-D), or a mixture of both (IBS-M) or none of these three characteristics (IBS-U ; U means "Unsubtype").

Preferably, the composition for use according to the invention is for treating IBS-C (constipation-predominant).

Preferably, the composition for use according to the invention is for treating subjects diagnosed according to ROME III criteria.
ROME is a process developed to classify the functional gastrointestinal disorders based on clinical symptoms.
The term "ROME III* criteria" is intended to mean criteria for irritable bowel syndrome as follows:
Recurrent abdominal pain or discomfort** at least 3 days per month in the last 3 months associated with 2 or more of the following:
   1. Improvement with defecation
   2. Onset associated with a change in frequency of stool
   3. Onset associated with a change in form (appearance) of stool
      * Criteria fulfilled for the last 3 months with symptom onset at least 6 months prior to diagnosis.
      * * "Discomfort" means an uncomfortable sensation not described as pain.

Other symptoms that are not essential but support the diagnosis of IBS:
- Abnormal stool frequency (greater than 3 bowel movements/day or less than 3 bowel movements/week);
- Abnormal stool form (lumpy/hard or loose/watery stool);
- Abnormal stool passage (straining, urgency, or feeling of incomplete bowel movement);
- Passage of mucus;
- Bloating or feeling of abdominal distension.

Some or all of IBS symptoms can occur at the same time - some symptoms may be more pronounced than others.

### DESCRIPTION OF THE FIGURES

FIGURE 1 describes the flow of patients through the protocol. From the 41 enrolled patients, 38 were randomised giving an ITT population of 38. Six patients have been excluded from the PP population (n=32).
FIGURE 2A : shows that the mean distension was lower in the product group vs control at the end of the product consumption period (-1.5; 95% CI (-3.3, 0.3); p=0.096). A trend (p=0.069) was also observed in PP population (-1.7; 95% CI (-3.6, 0.1). Data are expressed as mean girth distension (cm) ± SD. These data correspond to AUC values over these standardized 12h period for each of the 2 days of recording (pre and postintervention).
FIGURE 2B : Relative changes in maximum abdominal distension in ITT population. Data are expressed as relative change in maximal girth distension (cm) at the end of product comsumption vs baseline. Analysis of the difference between groups was done with Mann-Whitney-U test (* p<0.05).
FIGURE 3 A-B: Subjective bloating over a standardized period of 12h before starting product consumption (baseline test, A) and at the end of study (B) in ITT population. Data are expressed as mean bloating score from hour 1 to hour 13 allowing the determination of bloating score over the 13h period of the day. Bloating score was assessed with a 6-point likert scale (from none = 1 to very severe = 6).
FIGURE 3 C: Mean bloating score (AUC 24h) in ITT population. Data are expressed as bloating score ± SD. These data correspond to the mean bloating score over the standardized 13h period for each of the 2 days of recording (pre and post-intervention).
FIGURE 4 A-B: Subjective abdominal pain/discomfort over a standardized period of 12h before starting product consumption (baseline test, A) at the end of the study (B) in ITT population. Data are expressed as mean abdominal pain/discomfort score from hour1 to hour 13 allowing the determination of abdominal pain/discomfort score over the 13h period of the day. Abdominal pain/discomfort score was assessed with a 6-point likert scale (from none = 1 to very severe = 6).
FIGURE 4 C: Abdominal pain/discomfort score (AUC 24h) in ITT population. Data are expressed as abdominal pain/discomfort score ± SD. These data correspond to the mean abdominal pain/discomfort score over the standardized 13h period for each of the 2 days of recording (pre and post-intervention).
FIGURE 5: Abdominal bloating score over 4-week period of product consumption in ITT population. Data are expressed as mean weekly bloating score ± SD. Bloating score was assessed with a 6-point likert scale (from none=1 to very severe=6). Ancova test (* p<0.05).
FIGURE 6: Abdominal pain/discomfort score over 4-week period of product consumption in ITT population. Data are expressed as mean weekly abdominal pain/discomfort score ± SD. Abdominal pain/discomfort score was assessed with a 6-point likert scale (from none=1 to very severe=6).). Ancova test (* p<0.05).
FIGURE 7: Flatulence score over 4-week period of product consumption in ITT population. Data are expressed as mean weekly flatulence score ± SD. Flatulence score was assessed with a 6-point likert scale (from none=1 to very severe=6).). Ancova test (* p<0.05).
FIGURE 8: Overall IBS symptoms score over 4-week period of product consumption in ITT population. Data are expressed as mean weekly overall IBS symptoms score ± SD. Overall IBS symptoms score was assessed with a 6-point likert scale (from none=1 to very severe=6). Ancova test (* p<0.05).
FIGURE 9: Satisfaction with bowel habits over 4-week period of product consumption in ITT population. Data are expressed as percentage of patients satisfied with their bowel habits (slightly, moderately or completely) at each week. Chi-square test (** p<0.005; * p<0.05).
FIGURE 10: Overall well being over 4-week period of product consumption in ITT population. Data are expressed as percentage of patients satisfied with their overall well-being (slightly, moderately or completely) at each week.

### EXAMPLES

### Material and Methods

### Study population

The study was carried out in 38 female patients with IBS-C aged 18-70 years, diagnosed according to Rome III criteria (1) (19 per treatment group). Randomised patients who dropped out before the second AIP and transit assessment, as planned in the protocol were replaced in order to reach a total number of 34 patients with main criterion (i.e. abdominal distension) and transit assessment available for product efficacy analysis. Patients were recruited from the out patients departments of the University Hospitals of South Manchester (tertiary patients excluded), local general practices, advertisement in regional newspapers, and from an existing departmental volunteer pool of patients.
Patients fulfilled the Rome III criteria for IBS with predominant constipation based on the stool consistency pattern (1). Patients had to have a bowel frequency of at least 2 per week in order to exclude patients with severe constipation. Patients were excluded from the study if they had any significant illness other than IBS. Patients with history of laxative abuse were also excluded as well as patients taking antidepressive or analgesic drugs. Patients having taken antibiotics within 60 days prior the entry in the study were also excluded. Detailed inclusion and non inclusion criteria are described below:

### Inclusion Criteria

➢ Females between the ages of 18 and 70 years.
➢ Diagnostic criteria (Rome III)* for IBS-C will be as follows: Recurrent abdominal pain or discomfort** at least 3 days per month in the last 3 months associated with 2 or more of the following:
   - Improvement with defecation.
   - Onset associated with a change in frequency of stool.
   - Onset associated with a change in form (appearance) of stool.
   - Hard and lumpy stools (Bristol Stool scale 1 and/or 2) ≥ 25% and loose (mushy) or watery stools (Bristol Stool scale 6 and/or 7 - fluffy pieces with ragged edges, a mushy stool or watery, no solid pieces, entirely liquid) < 25% of bowel movements.***
   **Criteria fulfilled for the last 3 months with symptom onset at least 6 months prior to diagnosis. Only patients with 'active IBS' at the time of screening will be studied; defined as those who report pain*/*discomfort on at least 2 days of the baseline symptom diary assessment (see below)*.
   ***Discomfort means an uncomfortable sensation not described as pain.*
   ****In the absence of use of laxatives*.
➢ Patients who are able to communicate well with the investigator and to comply with the requirements for the entire study.
➢ Patients who provide written informed consent before participating in the study after being given a full description of the study.
➢ Patients of normal body weight or overweight ie. not obese (body mass index between 18 and 30 kg/m2).

### Exclusion criteria

➢ Any significant illness other than IBS will exclude patients from the study.
➢ Patients with severe constipation, defined as a bowel frequency of < 2 bowel movements per week. The patients must have a bowel frequency of at least 2 per week.
➢ Patients with evidence of cathartic colon or a history of laxative abuse, that in the Investigator's opinion is consistent with severe laxative dependence such that the patient is likely to require or use laxative during the study.
➢ Taking drugs that might modify gastrointestinal function.
➢ Taking antidepressive or analgesic drugs.
➢ Taking antibiotics within 60 days prior to entry into the study.
➢ Taking an investigational drug within the 30 days prior to entry into the study.
➢ Drinking alcohol above the recommended safe alcohol limit (<21 units/week).
➢ Fertile women* who are not currently taking oral birth control pills (at least 1 full monthly cycle prior to study medication administration and continued until 1 month following the last dose of study medication) should be using or complying with one of the other medically approved methods of contraception such as, but not exclusively, one of the following:
   - Intra-uterine device (IUD)
   - Double barrier methods (such as condoms and spermicide)
   - Abstinence, when in the opinion of the investigator, their occupation or life style gives sufficient evidence that abstinence will be maintained throughout the study and for 1 month thereafter. In the case of abstinence, it should be recorded in the source documents that the patient was appropriately counselled.
   **Fertile woman is defined as a woman who is not surgically sterile (i. e. hysterectomy, bilateral oophorectomy or bilateral tubal libation), or is not post-menopausal (a post-menopausal patient is defined as a patient who has not menstruated for 12 months or more). A urine pregnancy test will be performed at visit V1 and prior to each abdominal x-ray*.
➢ All medications and cigarette smoking will be prohibited for 48 hours prior to the study, whilst alcohol and caffeine containing drinks will be stopped for 24 hours before the study.
➢ Strenuous physical activity will be prohibited for 24 hours prior to the study.
➢ Patient with allergy or hypersensitivity to milk proteins.
➢ Patient having undergone a surgery in the month prior to inclusion.

Throughout the study, the patients had not to consume any probiotic or fermented dairy products other than those provided. They were encouraged to continue with all the other aspects of their dietary and physical exercise habits.

### Study design

The study was single-centre, randomized, double-blind, placebo-controlled, parallel-group in female patients with IBS-C assessing the effect of daily consumption of a fermented milk containing *B. *animalis* DN-173 010 (Activia®, test group) vs a control group.
The individual's participation has lasted approximately 50 days. The first 11 days were used to obtain baseline values for the outcome parameters. The 4 subsequent weeks (D0 to D28) have constituted the experimental phase. During this period, the patients had to consume 2 products per day (during D0 to D27). The products must be consumed with a meal twice a day, preferably once at 8am and once at 8pm (with the exception of the first day of consumption (t=0), where the patients were asked to consume the 2 pots with their evening meal (i.e. 8pm)). In the case of patients forgetting to eat the product they were advised to consume it with their next meal. They have ceased consumption of the study product at the end of week 4 (D27), and were then contacted by telephone on 7th day after last visit (D35).
The study protocol was conducted in accordance with the Declaration of Helsinki and approved by South Manchester Medical Research Ethics Committee. All subjects gave written informed consent before inclusion in the study.

### Products

The test product was a fermented milk (Activia®, Danone), containing *Bifidobacterium animalis* DN- 173 010 (1.25x10¹⁰ colony forming unit (cfu) per pot) together with the two classical yoghurt starters, *S. thermophilus* and *L. bulgaricus* (1.2x10⁹ cfu/pot). The test product was without flavour. The placebo control is a milk-based non-fermented dairy product without probiotics and with low content of lactose < 4 g/pot as in the test product. Each serving (one pot) of either test or placebo control product contained 125 g.

### Assessments and study endpoints

### Abdominal distension

Abdominal distension was measured using the technique of Abdominal Inductance Plethysmography (AIP) that has been described in detail elsewhere (13, 14-15) but briefly it works on the principle that a loop of wire forms an inductor, the inductance of which is dependent on the area enclosed by the loop. For the purposes of AIP, the wire is sewn into a band of elasticated fabric (approximately 8.5cm wide) in zig-zag fashion to allow for expansion (Respitrace inductive sensor, Ambulatory Monitoring Inc., New York, USA) and is worn like a belt around the abdomen. Attached to the wire is a small electronic circuit unit, which incorporates an inductor in a resonant circuit whose output frequency varies with the area enclosed by the band, and a small battery operated microprocessor "data logger" which records and stores the average frequency of the oscillator circuit for 30 seconds each minute. The data logger simultaneously records posture (standing, sitting and lying) via sealed mercury tilt switches (ASSEMtech Europe Ltd., Essex, UK) taped to the subject's chest and thigh. The cross sectional area of the abdomen recorded by the equipment is then converted into a circumferential measurement, as described previously.
As previous studies have shown that there is no statistically significant difference between girth measurements taken in the standing and sitting positions, girth whether in the sitting or standing position will be averaged over one hour periods throughout day 1.
With regards standardisation of record time, for each subject, if the length of record was different between the two evaluations, the shorter time was used as reference for the two records.
The length of record was specific to each subject and was thus not the same for all the subjects. It has varied from 11 to 14 hours.
AUC values, over these hourly data, for each of the two days of recording (pre and post intervention respectively) from the first hour after fitting the belt to the hour of retiring to bed in the evening, were calculated. The analysis of AUC were made on the same period, i.e. 12h period from hour 1 (baseline referenced as the beginning of the measurement at pre- or post- intervention sessions) to hour 13, in order to standardize the period of measurement for each patient and to have complete data for all time for all patients. This period corresponds to the minimal period with all AIP data available for all patients for pre- and post-intervention measurements. The main expression of this parameter was an incremental AUC.
In addition, mean abdominal girth from the beginning to end of day 1 referenced to the beginning of day 1 (i.e. mean abdominal girth from the second hour of the study to the end of day 1 minus mean girth for the first hour of the study) were determined.

Maximum distension was defined as the mean girth over one hour of recording at which girth was at its greatest. Whether maximal girth was associated with the end of day, meal ingestion or no specific identifiable event will be noted.

### Global assessment

The global assessment of IBS symptoms relief was done by one global question ("Do you consider that in the past week, you have had adequate relief of your IBS-symptoms (abdominal pain or discomfort, bloating or distension, altered bowel habit, overall well being) compared to the period before beginning the study yoghurt?"). If the answer to this question is yes, then the patient was asked to answer the following question: "How would you describe your relief?" "slight", "moderate", "a lot" or "complete". This assessment was done every 7th day after the start of the consumption of the study product.

### Satisfaction with Bowel Habit

The satisfaction with bowel habits or with overall wellbeing was assessed by answering to two independent questions ("How satisfied have you been with your bowel habit in the past week, compared to the period before beginning the study yoghurt?" and "How satisfied have you been with your overall well-being in the past week, compared to the period before beginning the study yoghurt?"). The answer to each question could be "not satisfied", "slightly satisfied", "moderately satisfied" or "completely satisfied". This assessment was done every 7th day after the start of the consumption of the study product.

### Statistical methods

The trial sample size calculation was based on previous data obtained on abdominal girth studies (18, 14-15). With 17 patients per group (34 patients in all), the study will have an 80% power to detect differences (mean abdominal girth from the beginning to the end of day 1 referenced to the beginning of day 1) of 2cm or more, assuming a common SD (16) of 2.0 and that a simple 2-sample t-test is used with the conventional 5% significance level. The randomisation was stratified by menstrual cycle/menopausal status Randomised patients who dropped out before the second AIP and transit time assessment were replaced in order to reach the number of 34 evaluable patients.
Two analyses will be carried out: one on the ITT population, and one on the PP population. The main analysis will be the analysis on the ITT. Baseline for criteria measured several times before study product consumption were the mean over the last 11 days before study product consumption, standardized for 7 days when needed. Age and body mass index (BMI) were taking into account as confounding factors in the covariate analysis according to data literature. Changes of parameters hourly assessed during 24-h periods (abdominal distension, i.e. AUC, mean, maximal, abdominal pain/discomfort, bloating, flatulence, overall IBS-symptom, bowel frequency, stool consistency, straining, urgency, incomplete evacuation) were compared between groups using analyses of covariance with baseline readings as covariate.
The main expression of abdominal distension is an incremental AUC over a standardized 12-h period which was considered as the main criterion. For daily assessment of parameters (recorded throughout the study in the dairies), a repeated measures analysis over 4-week was performed (time=week) as the main analysis (averaging over 7 days of recording where appropriate) to compare test and control groups. The analysis of the effect of each parameter at each week was performed with a ANCOVA, with appropriate adjustment for multiple testing, or a t-test if no covariate was taken into account. A longitudinal analysis over the time period using generalised estimating equations was also carried out.
IBS symptoms relief will be weekly assessed except for baseline time. First one with a binomial response ("Yes"/"No") and if it is "Yes" with a multinomial response. Satisfaction with bowel habits and with overall well-being were also assessed each week. A repeated measures analysis was performed (time=week) as the main analysis. The analysis of the effect at each week was performed with a ANCOVA, or logistic regression models as appropriate, with appropriate adjustment for multiple testing, or a t-test if no covariate is taken into account.

Changes in IBS severity score between groups were compared using analyses of covariance with baseline readings as covariate. Comparison of the changes for bowel transit (small and large) between the two groups was carried out using ANCOVA.

### Results

All results in tables and figures are the results on the ITT population. Results on PP population, when available, are given in the text following the description of the ITT results.

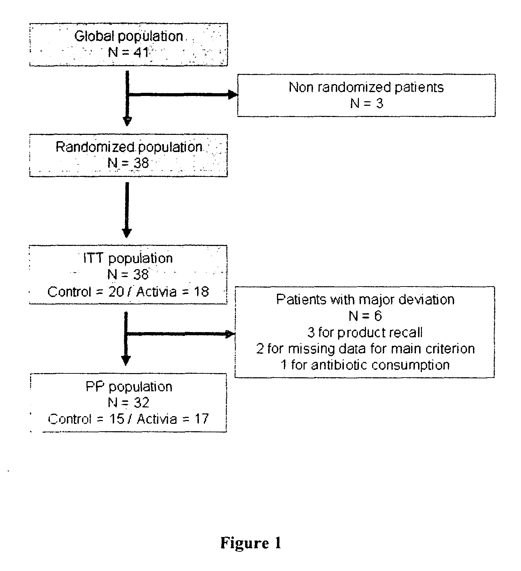

Figures 1 describes the flow of patients through the protocol.

### Abdominal distension

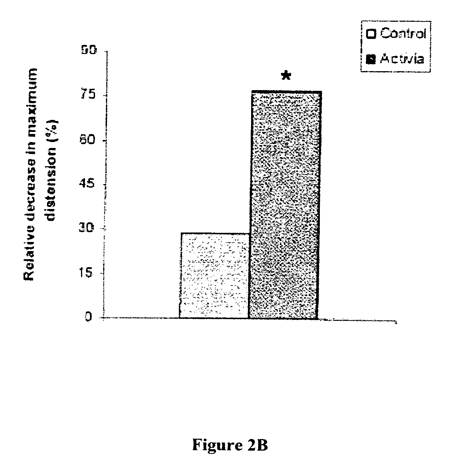

Changes in maximal abdominal distension are shown in figure 2B. There is a significant (p=0.029) larger percentage decrease in the relative maximum distension in the product group vs the control group, -77.1% vs -28.6 respectively. The effect was similar in the PP population (-77.1% vs -15.8%, p=0.022).

### IBS symptoms

### - 24-hour AUC measurements

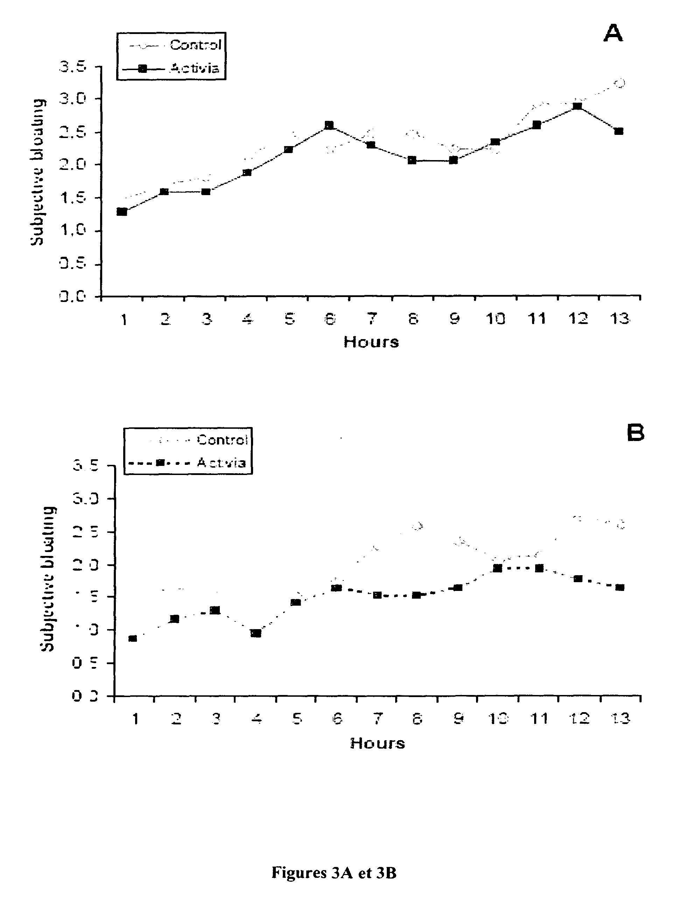

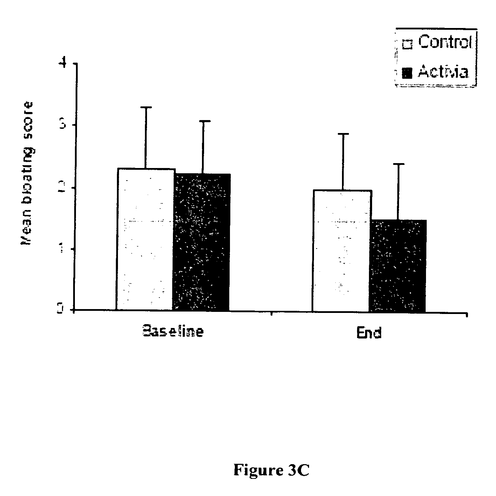

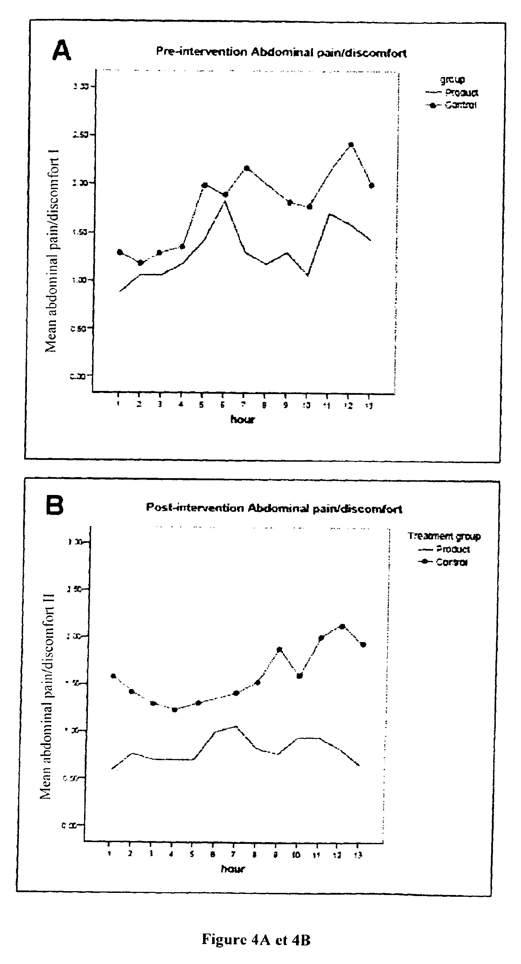

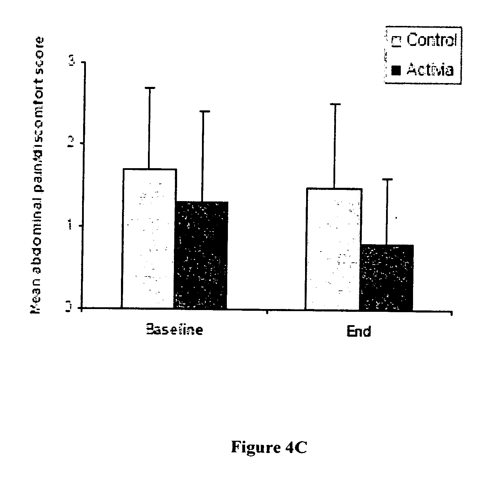

Results of the changes in abdominal bloating and abdominal pain/discomfort over the same period of recording of abdominal girth are shown in figures 3A-C and 4A-C, respectively.
The mean score of abdominal bloating was lower in the product group as compared to the control group at the end of product consumption period (-0.5; 95% CI (-1.0, 0.1); p=0.084) (figure 4C). This effect was significant in the PP population (-0.5; 95% CI (-1.0, 0.0); p=0.042).
This lower abdominal pain/discomfort score was significantly different than the one observed in control group (-0.6; 95% CI (-1.2, -0.1); p=0.024) in the PP population.

### - 4 week assessment

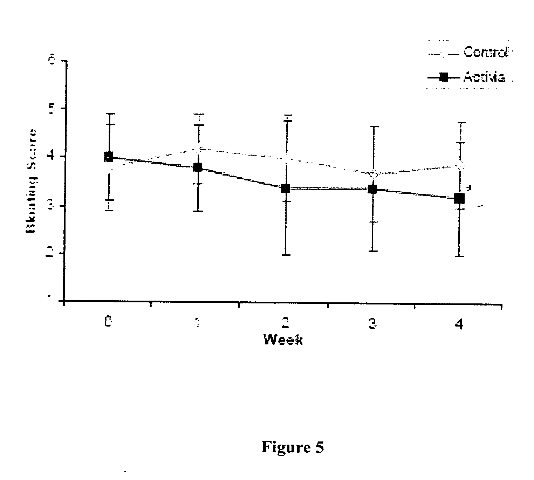

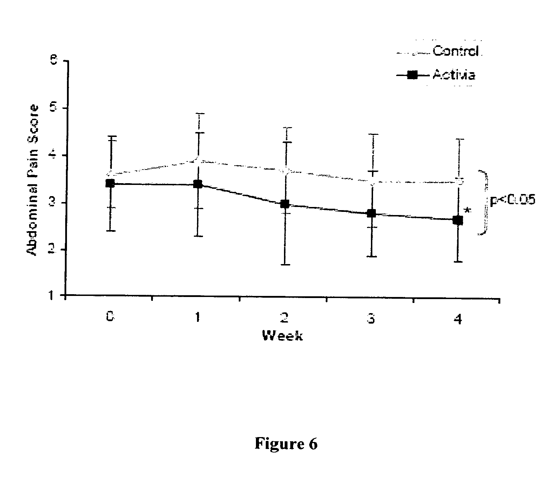

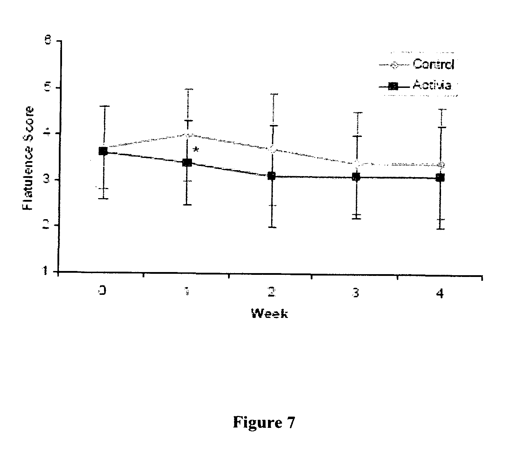

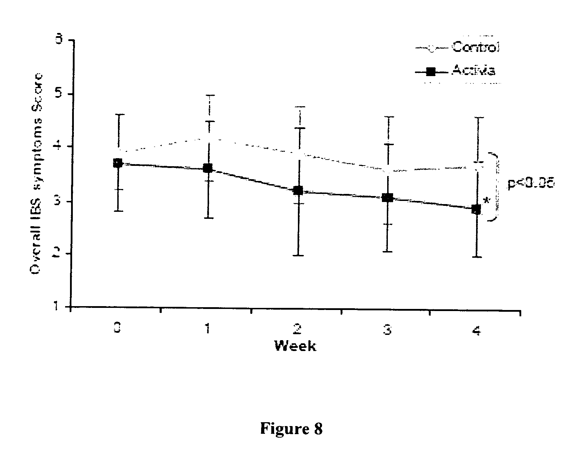

Results of the changes in abdominal bloating, abdominal pain/discomfort, flatulence and overall IBS symptoms over the 4-week period of product consumption are shown in figures 5, 6, 7 and 8, respectively.
Weekly analysis show that the improvement decrease in bloating score was significantly better (p=0.041, non-adjusted test) in product group at week 4 (Figure 5).
Abdominal pain/discomfort score was significantly (p=0.044) improved in the test group over the 4-week period of product consumption (mean overall difference between groups=-0.5; 95% (-1.0, 0.0) (figure 6). A significant improvement (p=0.039, non-adjusted test) is also observed in test group at the end of product consumption, i.e. week 4, when weekly analysis was performed.
Weekly analysis showed that the improvement decrease in flatulence score is significantly better (p=0.030, non-adjusted test) in product group at week 1 (Figure 7).
Overall IBS symptoms score was significantly (p=0.032) improved in the test group over the 4-week period of product consumption (mean overall difference between groups=-0.5; 95% (-1.0, -0.05) (figure 8). A significant improvement (p=0.031, non-adjusted test) was also observed in test group at the end of product consumption, i.e. week 4, when weekly analysis were done.

### Satisfaction symptoms relief

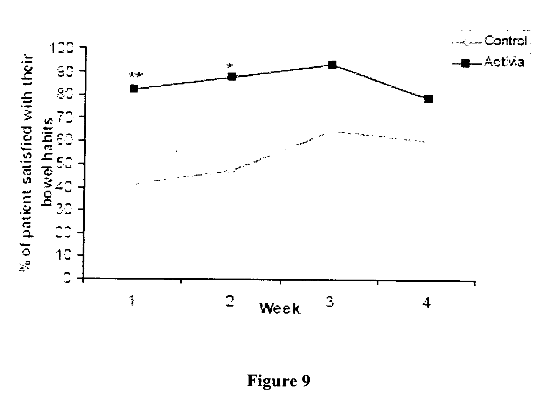

Weekly change of IBS symptoms relief showed significant higher rate of patients which were satisfied with their bowel habits (figure 9) at week 1 (82.4% (14/17 patients) in test group vs 41.2% (7/17 patients) in control group; p=0.001) and week 2 (87.5% (14/16 patients) in test group vs 47.1% (8/17 patients) in control group; p=0.026).

Results of the analysis on overall 4-week period are not available yet. The mean rate of patients satisfied with their bowel habits over the 4-week period of product consumption was 85% (14/17 patients) vs 53% (9/17 patients), for test and control groups respectively.

### Overall well being

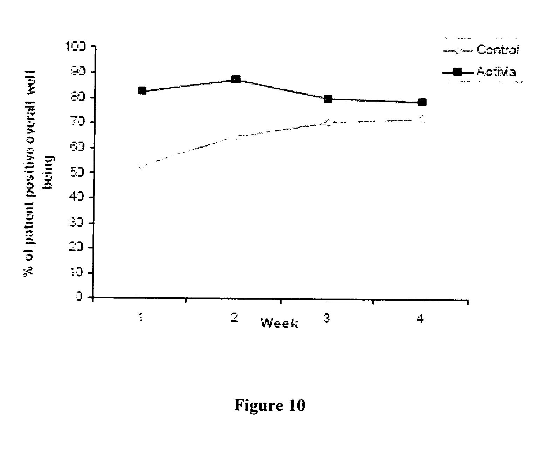

The mean rate of patients satisfied with their overall well being over the 4-week period of product consumption was 82% (12/15 patients) vs 65% (10/16 patients), for test and control groups respectively (figure 10).

### Conclusions

This study was designed to investigate the effect of the fermented dairy product containing the strain *B. animalis* DN-173 010 and a yoghurt symbiosis (i.e. Activia®) on abdominal distension that is a particular intrusive symptom in IBS. Global assessment of IBS symptoms were also done to determine the global effect of the product in a well characterised population of IBS women.
The present study showed that the consumption of a fermented dairy product containing the strain *B. animalis* DN-173 010 and a yoghurt symbiosis (i.e. Activia®) improves abdominal distension and as well as the overall global symptomatology of IBS (abdominal bloating, abdominal pain/discomfort, flatulence and overall IBS symptoms) in women with IBS-C.
Primary endpoint, abdominal distension, showed a significant larger percentage decrease in the relative maximum distension in the product group vs the control group. Indeed, a striking effect was observed in the decrease (-77%) of the maximal abdominal distension (relative expression). Interestingly, this effect on abdominal distension is associated with an improvement of both sensations of abdominal bloating and abdominal pain/discomfort during the same 12-h period of assessment.
Analysis on PP population, which could be considered as analysis of sensitivity, showed a significant improvement of abdominal bloating and abdominal pain/discomfort. The analysis of the 4-week period of product consumption demonstrated also beneficial effects on overall symptomatology of IBS as showed by the significant improvement of overall IBS symptoms over this period. This effect is supported by a significant reduction of abdominal pain/discomfort and a tendency to an improvement of abdominal bloating and flatulence.
Although for the evaluation of the clinical relevance the small sample size has to be kept in mind, global assessments of IBS-symptoms relief, satisfaction with bowel habits and with overall well-being are in favour of an important clinical change for the patients having consumed the product.

### References:

1. Longstreth GF, Thompson WG, Chey WD, Houghton LA, Mearin F, Spiller R. "Functional Bowel Disorders" ; Gastroenterology 2006; 130 (5): 1480-91.
2. Drossman DA, Li Z, Andruzzi E et al. "US Householder survey of functional gastrointestinal disorders: prevalence, sociodemography and health impact" ; Dig Dis Sci 1993, 38: 1569-1580.
3. Thompson WG. "A world view of IBS". In: Spiller R and Camilleri M, eds. "The Irritable Bowel Syndrome, Diagnosis and treatment" ; WB Saunders, 2002: 17-26.
4. Longstreth GF. "Definition and classification of IBS: current consensus and controversies" ; Gastroenterol Clin North Am 2005; 34: 173-187.
5. Longstreth GF, Bolus R, Naliboff B et al. "Impact of irritable bowel syndrome on patient lives: development and psychometric documentation of disease-specific measure for use in clinical trials" ; Eur J Gastroenterol Hepatol 2005; 17: 411-420.
6. Wilson A, Longstreth G, Knight K et al. "Quality of life in managed care patients with irritable bowel syndrome" ; Manage Care Interface 2004: 17: 24-28.
7. Spiller R, Aziz Q, Creed F, Emmanuel A, Houghton LA, Hungin P, Jones R, Kumar D, Rubin G, Trudghill N, Whorwell PJ. "Guidelines for the management of irritable bowel syndrome" ; Gut 2006 (in press).
8. Bougle et al. "Effect of Propionibacteria Supplementation on Fecal Bifidobacteria and Segmental Colonic Transit Time in Healthy Human Subjects" ; 1999 Scand. J. Gastroenterol. 34 p144.
9. Fioramonti J, Theodorou V and Bueno L. "Probiotics: what are they? What are their effects on gut physiology?" Best Pract Res Clin Gastroenterol 2003; 17:711-724.
9bis. Quigley EMM and Flourié B. Probiotics and irritable bowel syndrome: a rationale for their use and an assessment of the evidence to date. Neugastroenterol Motil 2007; 19:166-172.
10. US 20030147858
11. Agrawal A. and Whorwell P.J., "Abdominal bloating and distension in functional gastrointestinal disorders: epidemiology and exploration of possible mechanisms" ; Aliment Pharmacol Ther. 2007; 27:2-10.
12. Diplock et al. "Scientific concepts of functional foods in Europe: consensus document" ; British Journal of Nutrition, 1999, 81, S1-S27
13. Houghton LA, Lea R, Agrawal A, Reilly B and Whorwell PJ. "Relationship of abdominal bloating to distension in irritable bowel syndrome and effect of bowel habit" ; Gastroenterology 2006; 131:1003-1010.
14. Lewis M, Reilly B, Houghton LA et al. « Ambulatory abdominal inductance plethysmography: towards objective assessment of abdominal distension in irritable bowel syndrome" ; Gut 2001; 48: 216-20.
15. Reilly B, Bolton M, Houghton LA et al. « A device for 24 hour ambulatory monitoring of abdominal girth using inductive plethysmography" ; Physiol Meas 2002; 23: 661-70.
16 Agrawal A, Whorwell PJ, Houghton LA. "Is abdominal distension related to delayed small and large bowel transit in patients with constipation predominant irritable bowel syndrome ?" ; Gastroenterology 2006; 130 (No 4, Suppl 2) A93, 632.

## Claims

1. *Bifidobacterium animalis* for use in decreasing abdominal girth in an IBS
subject having or subjected to abdominal girth increase, wherein at least **1x10⁹** cfu of said *Bifidobacterium animalis* is administered per day to said subject during at least 15 days.

2. *Bifidobacterium animalis* for use according to claim 1. **characterized in that** the decrease of abdominal girth of the subject is at least about 1cm during the day.

3. *Bifidobacterium animalis* for use according to claim 1, **characterized in that** the decrease of abdominal girth is a decrease in the relative maximum abdominal distension.

4. *Bifidobacterium animalis* for use according to claim 3, **characterized in that** the decrease in the relative maximum abdominal distension is at least about 50%.

5. *Bifidobacterium animalis* for use according to any of claim 1 to 4, **characterized in that** said *Bifidobacterium animalis* is *Bifidobacterium animalis* deposited under the number CNCM I-2494.

6. *Bifidobacterium animalis* for use according to any of claim 1 to 5, **characterized in that** about 1,25x 10¹⁰ cfu of said bacteria is administered to the subject two times per day during at least 28 days.

7. *Bifidobacterium animalis* for use according to any of claim 1 to 6, **characterized in that** the subject is a female.

8. *Bifidobacterium animalis* for use according to any of claim 1 to 7, **characterized in that** said bacteria is administered in the form of a dairy product, preferably a fermented dairy product.

9. *Bifidobacterium animalis* for use according to claim 8, **characterized in that** the fermented dairy product is a yoghourt.

10. *Bifidobacterium animalis* for use according to any of claim 1 to 7, **characterized in that** said bacteria is administered in the form of a pharmaceutical composition

11. *Bifidobacterium animals* for use according to any of claim 1 to 10, **characterized in that** said use is for treating irritable bowel syndrome (IBS).

12. *Bifidobacterium animalis* for use according to claim 11, **characterized in that** said use is for treating IBS-C.

13. *Bifidobacterium animalis* for use according to claim 11 or 12 **characterized in that** said subject is diagnosed according to ROME III criteria.

14. Non-therapeutic Method for decreasing abdominal girth in a healthy subject comprising the step of administering to said subject at least about 1x 10⁹ cfu of a bacteria chosen in the group of bacteria of the *Bifidobacterium animalis* per day

15. Non therapeutic Method according to claim 14, **characterized in that** the bacteria is administered during at least 15 days.

16. Non therapeutic Method according to claim 14, **characterized in that** the decrease of abdominal girth of the subject is at least about 1 cm during the day.

17. Non therapeutic Method according to claim 14, **characterized in that** the decrease of abdominal girth is a decrease in the relative maximum abdominal distension.

18. Non therapeutic Method according to claim 17, **characterized in that** the decrease in the relative maximum abdominal distension is at least about 50%.

19. Non therapeutic Method according to claim 14 **characterized in that** the bacteria is *Bifidobacterium animalis* deposited under the number CNCM I-2494.

20. Non therapeutic Method according to any of claim 14 to 19 **characterized in that** about 1,25x 10¹⁰ cfu of said bacteria is administered to the subject two times per day during at least 28 days.

21. Non therapeutic Method according to any of claim 14 to 20 **characterized in that** the subject is a female.

22. Non therapeutic Method according to any of claim 14 to 21, **characterized in that** said bacteria is administered in the form of a dairy product, preferably a fermented dairy product.

23. Non therapeutic Method according to claim 22, **characterized in that** the fermented dairy product is a yoghourt.

## Patentansprüche

1. *Bifidobacterium animalis* zur Verwendung zur Verringerung des Bauchumfangs in einer Testperson mit IBS, die eine Zunahme des Bauchumfanges aufweist oder dem ausgesetzt ist, wobei mindestens 1 x 10⁹ KBU an *Bifidobacterium animalis* der Testperson für mindestens 15 Tage verabreicht wird.

2. *Bifidobacterium animalis* zur Verwendung nach Anspruch 1, **dadurch gekennzeichnet, dass** die Verringerung des Bauchumfanges der Testperson mindestens 1 cm während des Tages beträgt.

3. *Bifidobacterium animalis* zur Verwendung nach Anspruch 1, **dadurch gekennzeichnet, dass** die Verringerung des Bauchumfangs eine Verringerung der relativen maximalen Bauchdehnung ist.

4. *Bifidobacterium animalis* zur Verwendung nach Anspruch 3, **dadurch gekennzeichnet, dass** die Verringerung der relativen maximalen Bauchdehnung mindestens ungefähr 50 % ist.

5. *Bifidobacterium animalis* zur Verwendung nach einem der Ansprüche 1 bis 4, **dadurch gekennzeichnet, dass** das *Bifidobacterium animalis* ein unter der Nummer CMCM I-2494 hinterlegtes *Bifidobacterium animalis* ist.

6. *Bifidobacterium animalis* zur Verwendung nach einem der Ansprüche 1 bis 5, **dadurch gekennzeichnet, dass** ungefähr 1,25 x 10¹⁰ KBU des Bakteriums zweimal pro Tag für mindestens 28 Tage der Testperson verabreicht wird.

7. *Bifidobacterium animalis* zur Verwendung nach einem der Ansprüche 1 bis 6, **dadurch gekennzeichnet, dass** die Testperson weiblich ist.

8. *Bifidobacterium animalis* zur Verwendung nach einem der Ansprüche 1 bis 7, **dadurch gekennzeichnet, dass** das Bakterium in der Form eines Milchproduktes, bevorzugt eines fermentierten Milchproduktes verabreicht wird.

9. *Bifidobacterium animalis* zur Verwendung nach Anspruch 8, **dadurch gekennzeichnet, dass** das fermentierte Milchprodukt ein Joghurt ist.

10. *Bifidobacterium animalis* zur Verwendung nach einem der Ansprüche 1 bis 7, **dadurch gekennzeichnet, dass** das Bakterium in der Form einer pharmazeutischen Zusammensetzung verabreicht wird.

11. *Bifidobacterium animalis* zur Verwendung nach einem der Ansprüche 1 bis 10, **dadurch gekennzeichnet, dass** die Verwendung in der Behandlung eines Reizdarmsyndroms (IBS) besteht.

12. *Bifidobacterium animalis* zur Verwendung nach Anspruch 11, **dadurch gekennzeichnet, dass** die Verwendung in der Behandlung von IBS-C besteht.

13. *Bifidobacterium animalis* zur Verwendung nach Anspruch 11 oder 12, **dadurch gekennzeichnet, dass** die Testperson nach den Kriterien ROME III diagnostiziert wird.

14. Nicht-therapeutisches Verfahren zur Verringerung des Bauchumfangs in einer gesunden Testperson umfasssend die Schritte der Verabreichung von mindestens ungefähr 1 x 10⁹ KBU eines Bakteriums ausgewählt aus der Gruppe von Bakterien der *Bifidobacterium animalis* pro Tag an die Testperson.

15. Nicht-therapeutisches Verfahren nach Anspruch 14, **dadurch gekennzeichnet, dass** das Bakterium während für mindestens 15 Tagen verabreicht wird.

16. Nicht-therapeutisches Verfahren nach Anspruch 14, **dadurch gekennzeichnet, dass** die Verringerung des Bauchumfanges der Testperson mindestens ungefähr 1 cm während des Tages beträgt.

17. Nicht-therapeutisches Verfahren nach Anspruch 14, **dadurch gekennzeichnet, dass** die Verringerung des Bauchumfanges in einer Verringerung der relativen maximalen Bauchdehnung besteht.

18. Nicht-therapeutisches Verfahren nach Anspruch 17, **dadurch gekennzeichnet, dass** die Verringerung in der relativen maximalen Bauchdehnung mindestens ungefähr 50 % ist.

19. Nicht-therapeutisches Verfahren nach Anspruch 14, **dadurch gekennzeichnet, dass** das Bakterium ein unter Nummer CNCM I-2494 hinterlegtes *Bifidobacterium animalis* ist.

20. Nicht-therapeutisches Verfahren nach einem der Ansprüche 14 bis 19, **dadurch gekennzeichnet, dass** ungefähr 1,25 x 10¹⁰ KBU des Bakteriums der Testperson zweimal pro Tag für mindestens 28 Tagen verabreicht wird.

21. Nicht-therapeutisches Verfahren nach einem der Ansprüche 14 bis 20, **dadurch gekennzeichnet, dass** die Testperson weiblich ist.

22. Nicht-therapeutisches Verfahren nach einem der Ansprüche 14 bis 21, **dadurch gekennzeichnet, dass** das Bakterium in der Form eines Milchproduktes, bevorzugt eines fermentierten Milchproduktes, verabreicht wird.

23. Nichttherapeutisches Verfahren nach Anspruch 22, **dadurch gekennzeichnet, dass** das fermentierte Milchprodukt ein Joghurt ist.

## Revendications

1. *Bifidobacterium animalis* pour son utilisation pour réduire la circonférence abdominale chez un sujet atteint du syndrome du côlon irritable (IBS) présentant ou susceptible de présenter une augmentation de la circonférence abdominale, au moins 1 x 10⁹ cfu de ladite *Bifidobacterium animalis* étant administrée par jour au dit sujet pendant au moins 15 jours.

2. *Bifidobacterium animalis* pour son utilisation selon la revendication 1, **caractérisée en ce que** la réduction de la circonférence abdominale du sujet est d'au moins environ 1 cm pendant la journée.

3. *Bifidobacterium animalis* pour son utilisation selon la revendication 1, **caractérisée en ce que** la réduction de la circonférence abdominale correspond à une réduction de la distension abdominale maximale relative.

4. *Bifidobacterium animalis* pour son utilisation selon la revendication 3, **caractérisée en ce que** la réduction de la distension abdominale maximale relative est d'au moins environ 50 %.

5. *Bifidobacterium animalis* pour son utilisation selon l'une quelconque des revendications 1 à 4, **caractérisée en ce que** ladite *Bifidobacterium animalis* est une *Bifidobacterium animalis* déposée sous le numéro d'enregistrement CNCM 1-2494.

6. *Bifidobacterium animalis* pour son utilisation selon l'une quelconque des revendications 1 à 5, **caractérisée en ce qu'**environ 1,25 x 10¹⁰ cfu de ladite bactérie sont administrés au sujet deux fois par jour pendant au moins 28 jours.

7. *Bifidobacterium animalis* pour son utilisation selon l'une quelconque des revendications 1 à 6, **caractérisée en ce que** le sujet est une femme.

8. *Bifidobacterium animalis* pour son utilisation selon l'une quelconque des revendications 1 à 7, **caractérisée en ce que** ladite bactérie est administrée sous la forme d'un produit laitier, de préférence un produit laitier fermenté.

9. *Bifidobacterium animalis* pour son utilisation selon la revendication 8, **caractérisée en ce que** le produit laitier fermenté est un yaourt.

10. *Bifidobacterium animalis* pour son utilisation selon l'une quelconque des revendications 1 à 7, **caractérisée en ce que** ladite bactérie est administrée sous la forme d'une composition pharmaceutique.

11. *Bifidobacterium animalis* pour son utilisation selon l'une quelconque des revendications 1 à 10, **caractérisée en ce que** ladite utilisation est destinée à traiter le syndrome du côlon irritable (IBS).

12. *Bifidobacterium animalis* pour son utilisation selon la revendication 11, **caractérisée en ce que** ladite utilisation est destinée à traiter un IBS-C.

13. *Bifidobacterium animalis* pour son utilisation selon la revendication 11 ou 12, **caractérisée en ce que** ledit sujet est diagnostiqué selon les critères ROME III.

14. Méthode non thérapeutique de réduction de la circonférence abdominale chez un sujet sain comprenant l'étape consistant à administrer au dit sujet au moins environ 1 x 10⁹ cfu par jour d'une bactérie choisie dans le groupe de bactéries de la famille de *Bifidobacterium animalis.*

15. Méthode non thérapeutique selon la revendication 14, **caractérisée en ce que** la bactérie est administrée pendant au moins 15 jours.

16. Méthode non thérapeutique selon la revendication 14, **caractérisée en ce que** la réduction de la circonférence abdominale du sujet est d'au moins environ 1 cm pendant la journée.

17. Méthode non thérapeutique selon la revendication 14, **caractérisée en ce que** la réduction de la circonférence abdominale correspond à une réduction de la distension abdominale maximale relative.

18. Méthode non thérapeutique selon la revendication 17, **caractérisée en ce que** la réduction de la distension abdominale maximale relative est d'au moins environ 50 %.

19. Méthode non thérapeutique selon la revendication 14, **caractérisée en ce que** la bactérie est de la famille de *Bifidobacterium animalis* déposée sous le numéro d'enregistrement CNCM 1-2494.

20. Méthode non thérapeutique selon l'une quelconque des revendications 14 à 19, **caractérisée en ce qu'**environ 1,25 x 10¹⁰ cfu de ladite bactérie sont administrées au sujet deux fois par jour pendant au moins 28 jours.

21. Méthode non thérapeutique selon l'une quelconque des revendications 14 à 20, **caractérisée en ce que** le sujet est une femme.

22. Méthode non thérapeutique selon l'une quelconque des revendications 14 à 21, **caractérisée en ce que** ladite bactérie est administrée sous la forme d'un produit laitier, de préférence un produit laitier fermenté.

23. Méthode non thérapeutique selon la revendication 22, **caractérisée en ce que** le produit laitier fermenté est un yaourt.
